# EUROPEAN PATENT SPECIFICATION

(11) **EP 0 717 109 B1**
(45) Date of publication and mention of the grant of the patent: **12.01.2005**
(21) Application number: 95114545.7
(22) Date of filing: 21.11.1989
(51) Int. Cl.: C12N 15/48, C07K 14/16, C12N 15/62, G01N 33/569

(54) **Synthetic DNA derived recombinant HIV-2 antigens**
Rekombinante HIV-2 Antigenen, abgeleitete aus synthetischen DNS
Antigènes recombinants de HIV-2 dérivés d'ADN synthétique

(30) Priority: 23.11.1988 US 275309
(43) Date of publication of application: 19.06.1996
(62) Divisional of application: 89121513.9
(73) Proprietor: Abbott Laboratories, Abbott Park, Illinois 60064-3500 (US)
(72) Inventor: Devare, Sushil G., Northbrook, Illinois 60062 (US); Casey, James M., Gurnee, Illinois 60031 (US); Desai, Suresh M., Libertyville, Illinois 60048 (US)
(74) Representative: Modiano, Guido, Dr.-Ing.

(56) References cited:
- EP-A- 0 001 931
- EP-A- 0 187 041
- EP-A- 0 199 301
- EP-A- 0 331 961
- EP-A- 0 400 245
- WO-A-88/03562
- WO-A-88/05440
- NATURE, vol. 326, no. 6114, 16 April 1987 LONDON GB, pages 662-669, GUYADER, M. ET AL. 'Genome organization and transactivation of the human immunodeficiency virus type 2'
- GENE, vol. 45, no. 3, 1986 AMSTERDAM NL, pages 317-325, KELLEY, K.A. ET AL. 'Synthesis of fusion and mature murine alpha interferons in Escherichia coli'

## Description

### BACKGROUND OF THE INVENTION

The present invention relates to recombinant HIV (Human Immunodeficiency Virus) antigens. Recombinant antigens derived from the molecular cloning and expression in a heterologous expression system of the synthetic DNA sequences of the various HIV antigens can be used as reagents for the detection of antibodies and antigen in body fluids from individuals exposed to various HIV isolates.

The nucleotide sequence of the proviral genome has been determined for several HIV isolates, including HIV-1 strains HTLV-III (Ratner et al., *Nature* (1985) 313:277); ARV-2 (Sanchez-Pescador et al., *Science* (1985) 227:484); LAV (Wain-Hobson et al., *Cell* (1985) 40:9); and CDC-451 (Desai et al., *Proc. Natl. Acad. Sci*. *USA* (1986) 83:8380). The nucleotide sequence of the HIV-2 ROD isolate was reported by Guyader et al. (*Nature* (1987) 326:662).

HIV antigens have been obtained from the virus grown in tissue culture, or from a molecularly cloned genomic fragment expressed in heterologous hosts such as Escherichia coli. The tissue culture derived virus involves the cumbersome and often difficult process of growing virus infected cells in stringent sterile conditions. Further, the virus derived from tissue culture is infectious, and, therefore is hazardous to the health of individuals involved in propagation and purification. The expression of molecularly cloned HIV genomic fragments overcomes the biohazard problem. Generally, an HIV genomic fragment from a single HIV isolate with mammalian codons is expressed in a heterologous system, such as, bacteria or yeast, and is limited to the use of available restriction sites present in the viral genome for cloning and expression.

It has been difficult to obtain expression in heterologous systems of some of the HIV proteins, such as the HIV-1 envelope antigen gp41. Several researchers have tried deleting the hydrophobic regions of the HIV-1 gp41 to increase expression levels. UK Patent Application GB 2188639 discloses an HTLV-III gag/env gene protein wherein the env fragment of the DNA sequence deleted codons corresponding to the first hydrophobic region of the gp41 protein. U.S. Patent No. 4,753,873 discloses a peptide fragment that is encoded by a nucleotide sequence wherein the nucleotides coding for a first and second hydrophobic region of HTLV-III gp41 are deleted.

Poor expression can be the result of many factors, including the specific nucleic acid sequence of the gene to be expressed, the mammalian codons of the gene sequence to be expressed may not be efficiently transcribed and translated in a particular heterologous system, and the secondary structure of the transcribed messenger RNA. The use of synthetic DNA fragments can increase expression in heterologous systems.

### SUMMARY OF THE INVENTION

Recombinant antigens which are derived from the molecular cloning and expression of synthetic DNA sequences in heterologous hosts are provided. Synthetic DNA sequences coding for the recombinant antigens of the invention are further provided. The synthetic DNA sequences selected for expression of various HIV antigens are based on the amino acid sequence of either a single isolate or several isolates, optimized for expression in Escherichia coli by specific codon selection. The synthetic DNA sequence gives higher expression of the particular antigen encoded. These antigens can be substituted for viral antigens derived from tissue culture for use as diagnostic and therapeutic reagents.

The present invention can be utilized to synthesize full length HIV transmembrane envelope gene using bacterial codons. Another aspect of the invention involves the linkage of sequences which are poorly expressed as individual proteins, to sequences which are expressed with high efficiency. The combination of the sequence of the entire coding region of a gene of one virus with coding sequences of another gene from a different virus to produce a fusion protein can be achieved. The fusion proteins thus expressed have a unique advantage of antigenic epitopes of two viral antigens.

The present invention includes full length synthetic genes (FSG) for HIV-2 transmembrane glycoprotein (TMP).

### DESCRIPTION OF THE DRAWINGS

Fig. 1 illustrates the DNA and amino acid sequence of the full length synthetic HIV-2 TMP, indicating restriction enzymes used to assemble the gene including linker sequences at both ends to facilitate cloning.
Fig. 2 illustrates the three major subfragments used to construct the synthetic HIV-2 TMP gene.
Fig. 3 is a schematic diagram of the assembly of the major subfragments to form the full length synthetic HIV-2 TMP and its cloning into pUC8 to generate pJC28.
Fig. 4 is a schematic diagram of the cloning of synthetic HIV-2 TMP fragment A into pUC19 to generate pJC22 and into pTB210 to generate pJC100.
Fig. 5 is a schematic diagram of the cloning of synthetic HIV-2 TMP into lambda pL expression vectors to generate pSD306 and pSD307.
Fig. 6 indicates the specific amino acid sequences of pL constructs pSD306 and pSD307, indicating all linker sequences, HIV-1 gag sequences, and HIV-2 TMP sequences.
Fig. 7 illustrates results of expression analysis of pSD306 in *E. coli* CAG456 cells. A) Coomassie stained gel; B) Immunoblot using HIV-2 positive human sera.
Fig. 8 illustrates results of expression analysis of pSD307 in *E. coli* pRK248.clts/RR1 cells. A) Coomassie stained gel; B) Immunoblot using HIV-2 positive human sera.

### DETAILED DESCRIPTION OF THE INVENTION

Synthetic DNA fragments of the HIV genome can be synthesized based on their corresponding amino acid sequences. By comparing the particular region of interest between different isolates, a sequence can be selected which is different from any sequence that exists in nature, because the sequence is a compilation of the sequences from various isolates.

Other properties can be built into the sequence. For example, codons can be switched for optimal expression in bacteria or yeast, specific restriction sites can be introduced, and other restriction sites can be removed. In addition, the sequence should have specific restriction sites at both 5' and 3' ends of the fragment to facilitate cloning in a particular vector. Synthetic DNA fragments can be synthesized as follows: (1) select an unique protein sequence, (2) reverse translate to determine complementary DNA sequence, (3) optimize codons for bacterial or yeast expression, and (4) introduce and/or remove specific restriction sites.

Sixty-one distinct nucleotide codons code for 20 amino acids giving rise to the degeneracy in the genetic code. Researchers have reported the frequencies of codons used in nucleic acids for both eukaryotic and prokaryotic organisms. (Grantham et al., *Nucleic Acids Res.* [1980] 9:r43; Gouy et al., *Nucleic Acids Res.* [1982] 10:7055; Sharp et al., *Nucleic Acids Res*. [1986] 14:7737.) Sequences from the entire *E. coli* genome, with examples of sequences from the chromosome, transposons, and plasmids, have been analyzed. These sequences code for structural proteins, enzymes and regulatory proteins. Correlation has been shown between the degree of codon bias within a particular gene and the level of gene expression.

It is preferred that the same codon triplet for each particular amino acid of the synthetic DNA sequence be used. However, alternative codon(s) can be used to add or delete a particular restriction site. The sequence should include unique restriction sites which can be used to delete a specific fragment or sequence, or substitute a particular sequence in case of an error in the original synthesis.

Vector systems which can be used include plant, bacterial, yeast, insect, and mammalian expression systems. It is preferred that the codons are optimized for expression in the system used. The proteins and polypeptides provided by the invention, which are cloned and expressed in heterologous systems, as described above, can be used for diagnostic and therapeutic purposes.

A preferred expression system utilizes the lambda pL vector system. This expression system has the following features: (1) a strong lambda pL promoter, (2) a strong three-frame translation terminator rrnBt1, and (3) translation starts at an ATG codon, eight base pairs from the ribosome binding site located within an accessible Ncol restriction site.

Another advantage of the expression system of the present invention is that one can customize the synthetic DNA fragments so they contain specific DNA sequences which express proteins with desired amino acid sequences, and further allows one the capability of adding, at either the 5' or 3' end, other DNA sequences to facilitate the transfer of synthetic fragments into various vectors.

Additionally, the use of particular restriction sites at both ends of the fragment may also facilitate incorporation of the fragment into other sequences to generate fusion proteins, which can also be used as diagnostic and therapeutic reagents, which can be used, for example, in a single assay screening system for the detection of both AIDS and Hepatitis B in prospective blood donors. Alternatively, the assay can be used to track the course of a patient's infection.

Other proteins from any source, including bacterial, yeast, insect, plant or mammalian, can be used with the synthetic DNA fragments of the invention to produce fusion proteins. Those which are expressed efficiently in their respective expression systems are especially preferred because they can enhance the expression of the synthetic fragment of the fusion protein.

The synthetic DNA sequences of the present invention, derived from several HIV isolates and optimized for expression in *E. coli*, provides continuous availability and uniformity of HIV antigen preparations which will recognize test sera from individuals exposed to genetically distinguishable variant HIV isolates. The recombinant antigen expression is very stable since *E. coli* codons have been used for its synthesis. Moreover, the insertion of specific restriction sites allows addition, deletion, or substitution in important antigenic epitopes in the coding sequences, a property difficult to achieve when naturally occurring HIV sequences are utilized for expression. Construction of synthetic genes also allows the addition of protein sequences at either amino- or carboxyl- terminus of the gene thereby allowing the development of novel reagents. For example,
the HIV-1 core antigen DNA sequence can be fused to the HIV-2 gp41 sequences, both of which can be expressed at high levels in heterologous host systems such as *E. coli*.

An *E. coli* strain containing a plasmid useful for constructs of the invention have been deposited at the American Type Culture Collection, Rockville, Maryland, on November 22, 1988, under the accession no. ATCC 67856 (pSD306/CAG456).

The following examples further describe the invention. The examples are not intended to limit the invention in any manner.

### Reagents and enzymes

Media such as Luria-Bertani (LB) and Superbroth II (Dri Form) were obtained from Gibco Laboratories Life Technologies, Inc., Madison, Wisconsin. Restriction enzymes, Klenow fragment of DNA polymerase I, T4 DNA ligase, T4 polynucleotide kinase, nucleic acid molecular weight standards, M13 sequencing system, X-gal (5-bromo-4-chloro-3-indonyl-β-D-galactoside), IPTG (isopropyl-β-D-thiogalactoside), glycerol, Dithiothreitol, 4-chloro-1-napthol were purchased from Boehringer Mannheim Biochemicals, Indianapolis, Indiana; or New England Biolabs, Inc., Beverly, Massachusetts; or Bethesda Research Laboratories Life Technologies, Inc., Gaithersburg, Maryland. Prestained protein molecular weight standards, acrylamide (crystallized, electrophoretic grade >99%); N-N'-Methylene-bis-acrylamide (BIS); N,N,N',N',-Tetramethylethylenediamine (TEMED) and sodium dodecylsulfate (SDS) were purchased from BioRad Laboratories, Richmond, California. Lysozyme and ampicillin were obtained from Sigma Chemical Co., St. Louis, Missouri. Horseradish peroxidase (HRPO) labeled secondary antibodies were obtained from Kirkegaard & Perry Laboratories, Inc., Gaithersburg, Maryland. Seaplaque agarose (low melting agarose) was purchased from FMC Bioproducts, Rockland, Maine.

T50E10 contained 50 mM Tris, pH 8.0, 10 mM EDTA; 1X TG contained 100 mM Tris, pH 7.5 and 10% glycerol; 2X SDS/PAGE loading buffer consisted of 15% glycerol, 5% SDS, 100 mM Tris base, 1M β-mercaptoethanol and 0.8% Bromophenol blue dye; TBS contained 50 mM Tris, pH 8.0, and 150 mM sodium chloride; Blocking solution consisted of 5% Carnation nonfat dry milk in TBS.

### Host cell cultures, DNA sources and vectors

*E. coli* JM103 cells, pUC8, pUC18, pUC19 and M13 cloning vectors were purchased from Pharmacia LKB Biotechnology, Inc.. Piscataway, New Jersey; Competent Epicurean™ coli strains XL1-Blue and JM109 were purchased from Stratagene Cloning Systems, La Jolla, California. RR1 cells were obtained from Coli Genetic Stock Center, Yale University, New Haven, Connecticut; and *E. coli* CAG456 cells from Dr. Carol Gross, University of Wisconsin, Madison, Wisconsin. Vector pRK248.clts was obtained from Dr. Donald R. Helinski, University of California, San Diego, California.

### General methods

All restriction enzyme digestions were performed according to suppliers' instructions. At least 5 units of enzyme were used per microgram of DNA, and sufficient incubation was allowed to complete digestions of DNA. Standard procedures were used for mini cell lysate DNA preparation, phenol-chloroform extraction, ethanol precipitation of DNA, restriction analysis of DNA on agarose, and low melting agarose gel purification of DNA fragments (Maniatis et al., *Molecular Cloning. A Laboratory Manual* [New York: Cold Spring Harbor, 1982]). Plasmid isolations from *E. coli* strains used the alkali lysis procedure and cesium chloride-ethidium bromide density gradient method (Maniatis et al., supra). Standard buffers were used for T4 DNA ligase and T4 polynucleotide kinase (Maniatis et al., supra).

### EXAMPLES

### Example 1

### Synthesis and cloning of synthetic HIV-2 TMP and fragment thereof

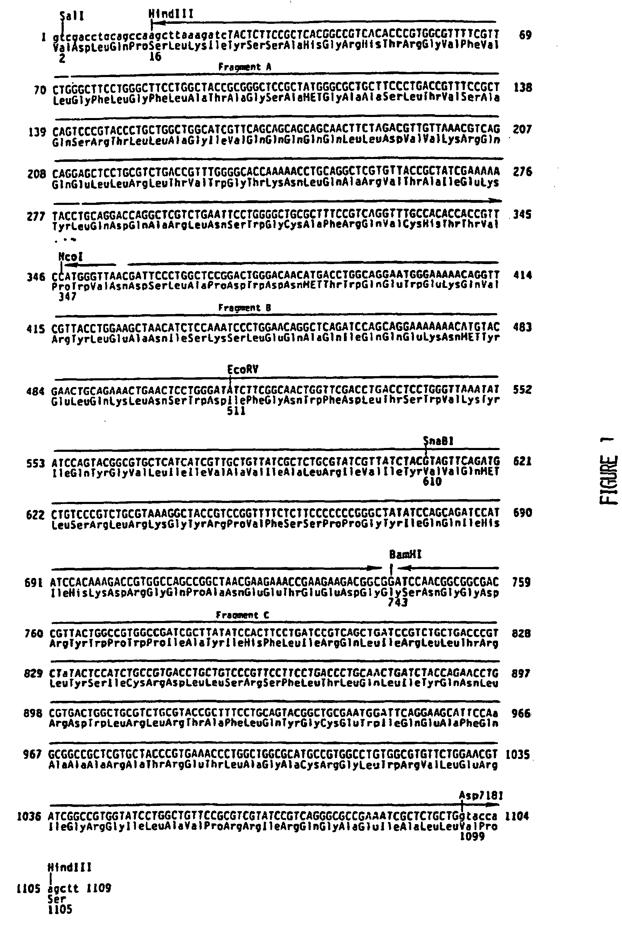

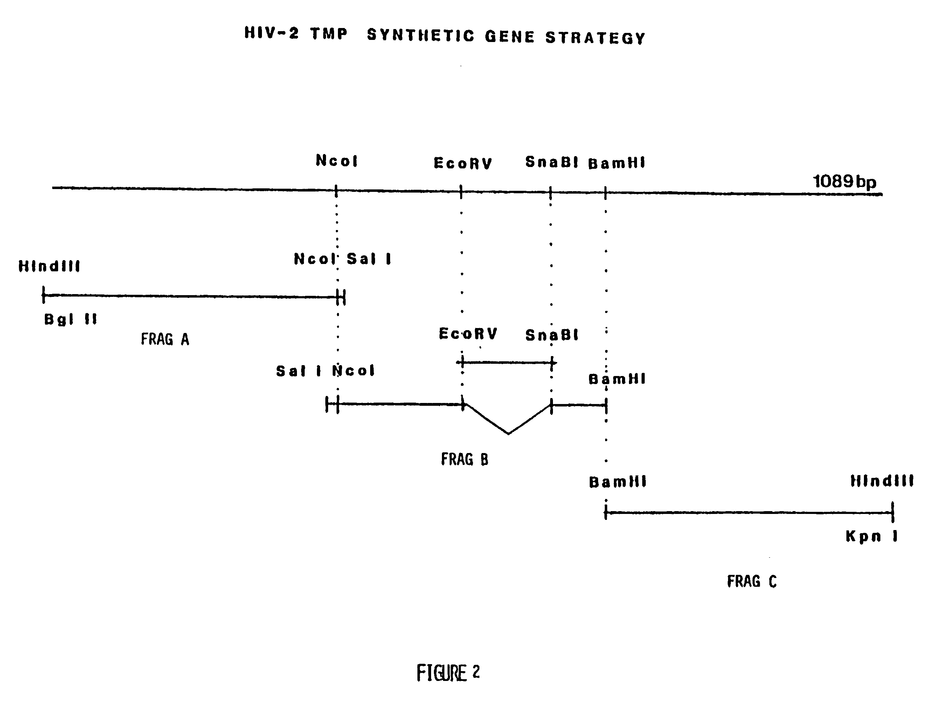

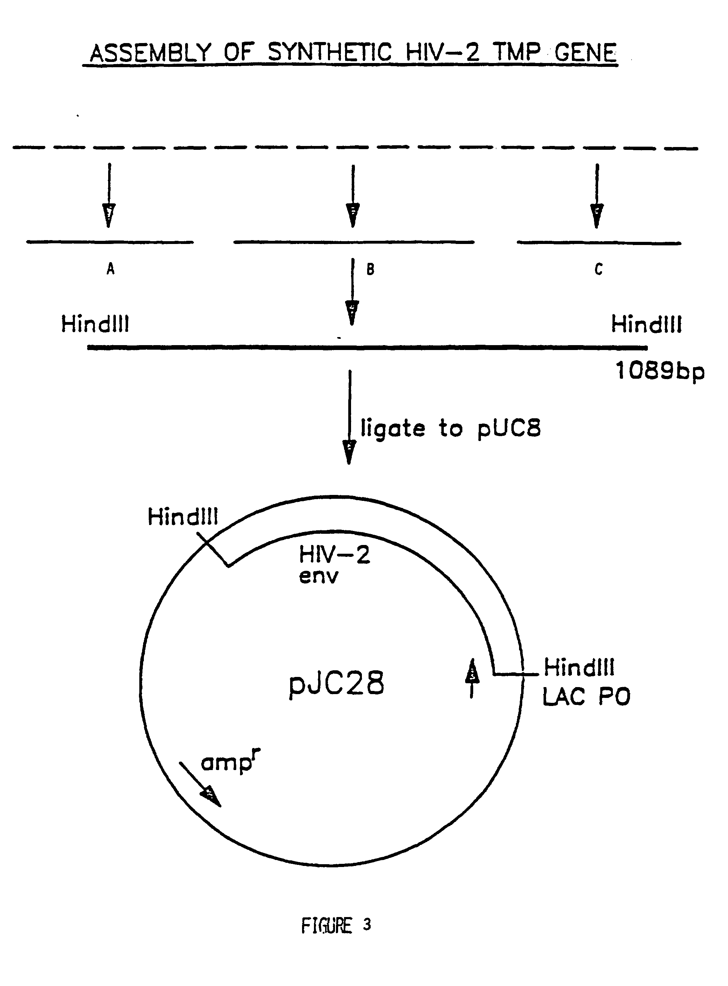

The entire HIV-2 transmembrane protein (TMP) was chemically synthesized using the method of oligonucleotide directed double-stranded break repair disclosed in U.S. Patent Application Serial No. 883,242, filed July 8, 1986 by Mandecki (EPO 87109357.1), which is incorporated herein by reference. Envelope amino acid residues 502-858 of the HIV-2 ROD isolate (numbering by Guyader et al., supra) were reverse translated using codon assignments optimal for expression in *E. coli*. After specific nucleotides were assigned to the remaining ambiguous nucleotides, as previously described, the full length TMP sequence was generated as indicated in Fig. 1. The synthetic gene was assembled and cloned as three separate subfragments represented by fragment A, a 335 bp HindIII-NcoI fragment, fragment B, a 309 bp NcoI-BamHI fragment (29 hydrophobic amino acid residues deleted), and fragment C, a 362 bp BamHI-Hindlll fragment, as depicted in Fig. 2. A fourth fragment containing the deleted twenty-nine hydrophobic amino acid residues was cloned into the 309 bp Ncol-BamHl fragment as an EcoRV-SnaBI fragment (Fig. 2). The three major subfragments were cloned into pUC vectors, transformed into JM109 cells and their primary nucleotide sequences confirmed, as previously described. The fragments were then gel purified and ligated together to form the 1089 bp full length synthetic HIV-2 TMP. This 1089 bp HindIII fragment was cloned into pUC8 and designated pJC28 (Figure 3 ).

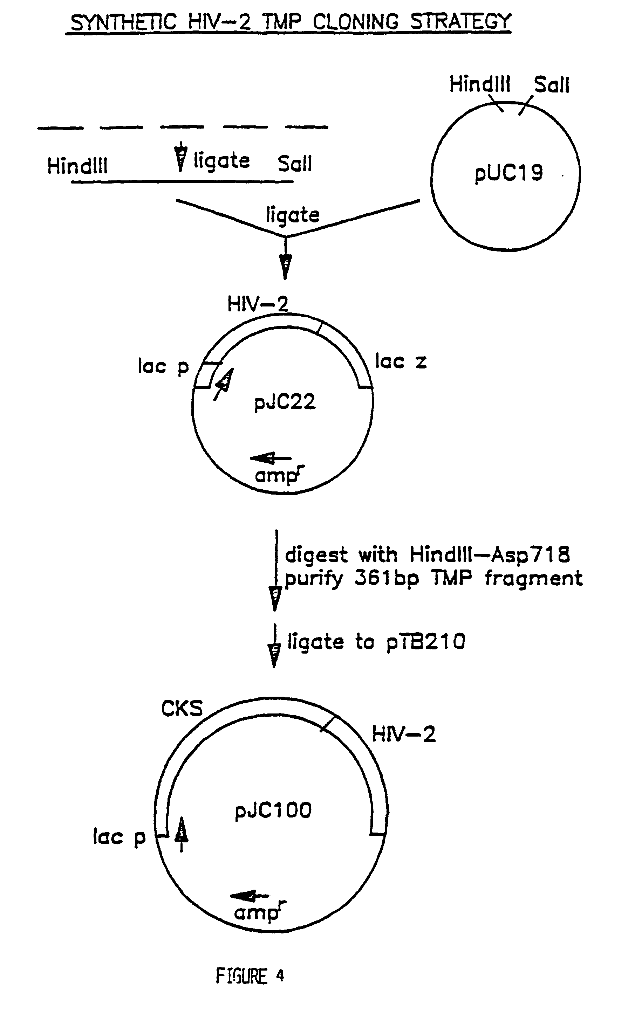

Fragment A encoding the amino terminal 108 amino acids of HIV-2 TMP (from Tyr 502 to Trp 609 [Guyader et al., supra]) was cloned at the HindIII-Sall sites of pUC19. A clone, designated pJC22, was identified by restriction mapping and its primary nucleotide sequence was confirmed. Plasmid pJC22 was digested with HindIII-Asp718 to release a 361 bp fragment containing the synthetic HIV-2 TMP gene fragment which was ligated into the HindIII-Asp718 sites of plasmid pTB210 and transformed into *E. coli* XL1 cells. Plasmid pTB210 is disclosed in a U.S. Patent Application entitled "CKS Method of Protein Synthesis", concurrently filed by T. Bolling et al., which is a CIP of an earlier application, U.S. Serial No. 167,067, filed March 11, 1988, which is hereby incorporated by reference. A clone, designated pJC100 (Fig.4 ), was isolated and restriction mapped to identify the hybrid gene of kdsB (encoding CKS) and HIV-2 TMP fragment.

### Example 2

### Cloning of synthetic HIV-2 TMP in lambda pL vectors

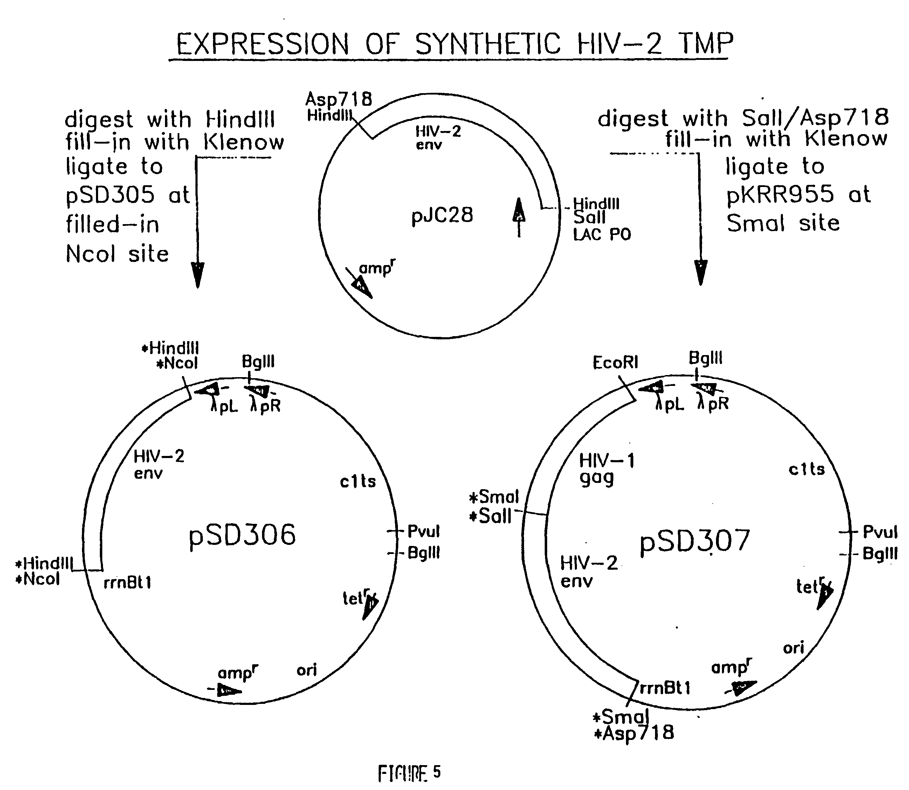

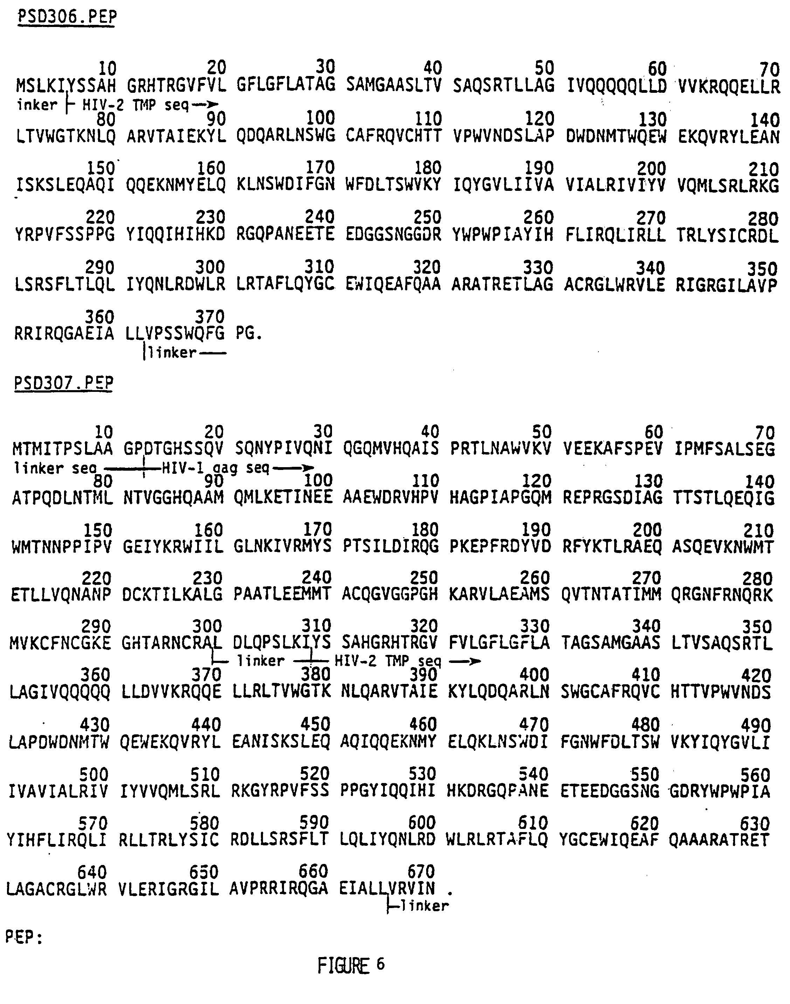

The 1089 bp HindIII fragment containing the entire HIV-2 TMP was isolated from pJC28, filled in with the Klenow fragment of DNA polymerase I to produce blunt ends and cloned directly behind an ATG start codon provided by the filled in Ncol site of pSD305 (pSDKR816 with clts inserted. Vector pSDKR816 contains the strong lamda pL promoter and the temperature sensitive cl repressor gene described in Benard et al., Gene (1979) 5:59. Such vector is a derivative of pBR322, containing a lambda pL promoter and a synthetic Shine-Dalgarno sequence, followed by a restriction site used for cloning various genes of interest. In addition, this vector contains the strong three-frame translation terminator rrnBt1. Vector pSDKR816 contains a Ncol restriction site which provides an ATG start codon optimally spaced from the start of transcription. Similarly, an 1097 bp Sall-Asp718 fragment containing the entire HIV-2 TMP was isolated from pJC28, filled in with the Klenow fragment of DNA polymerase I to produce blunt ends and cloned at the Smal site of pKRR955 (Vector containing the strong lambda pL promoter and the temperature sensitive cl repressor gene described in Benard et al. Gene (1979) 5:59. Such vector is a derivative of pBR322, containing a lambda pL promoter and a synthetic Shine-Dalgarno sequence, followed by a restriction site used for cloning various genes of interest. In addition, this vector contains the strong three-frame translation terminator rrnBt1.) to produce an HIV-1 gag/HIV-2 TMP fusion protein. The clone containing the HIV-2 TMP gene under control of the lambda pL promoter was designated pSD306 and the clone containing the HIV-2 TMP as a fusion to HIV-1 gag under control of the lambda pL promoter was designated pSD307, as outlined in Fig. 5. After transformation of pSD306 into E.coli CAG456 cells (Baker, PNAS (1984) 81:6779) and pSD307 into E.coli pRK248.clts/RR1 cells, single cell clones were isolated and restriction mapped to demonstrate the presence and orientation of the HIV-2 TMP gene. The specific amino acid sequences of pSD306 and pSD307 are presented in Fig. 6, indicating linker derived sequences, HIV-1 gag sequences and HIV-2 TMP sequences. Expression of the synthetic HIV-2 TMP gene was induced in these cultures by temperature shift methods (cells were grown in Superbroth II media at 30°C to an OD600 of 0.5, at which time the cultures were shifted to 42°C to inactivate the temperature sensitive cl repressor and thereby induce expression off the lambda pL promoter). Aliquots of the cultures before and after induction were subjected to SDS/PAGE analysis for both Coomassie blue staining and immunoblotting using HIV-2 positive human sera, as follows.

The cells were pelleted, then resuspended in either 1X TG buffer or T50E10 buffer. An equal volume of 2X SDS/PAGE loading buffer was added to the 1X TG suspended cells to produce the whole lysate. The sample resuspended in T50E10 was sonicated eight times for 30 seconds each, at a power setting of 10 watts, using the microtip provided with the Vibra Cell Sonicator (Sonics and Materials, Inc., Danbury, CT). The sonicated sample was then centrifuged to remove the insoluble fraction which was resuspended in the original starting volume of T50E10. An equal volume of 2X SDS/PAGE loading buffer was added to both the sonicated soluble and insoluble fractions, which together with the whole cell lysate, were boiled for 5 min, centrifuged to remove any remaining insoluble material, and aliquots (15µl) were separated on duplicate 12.5% SDS/PAGE gels. Proteins from one such gel were electrophoretically transferred to nitrocellulose for immunoblotting with AIDS patients' sera, as previously described. The second gel was fixed in a solution of 50% methanol, 10% acetic acid for twenty minutes at room temperature, and then stained with 0.25% Coomassie blue dye in a solution of 50% methanol, 10% acetic acid for 30 minutes. Destaining was carried out using a solution of 10% methanol, 7% acetic acid for 3-4 hr, or until a clear background was obtained.

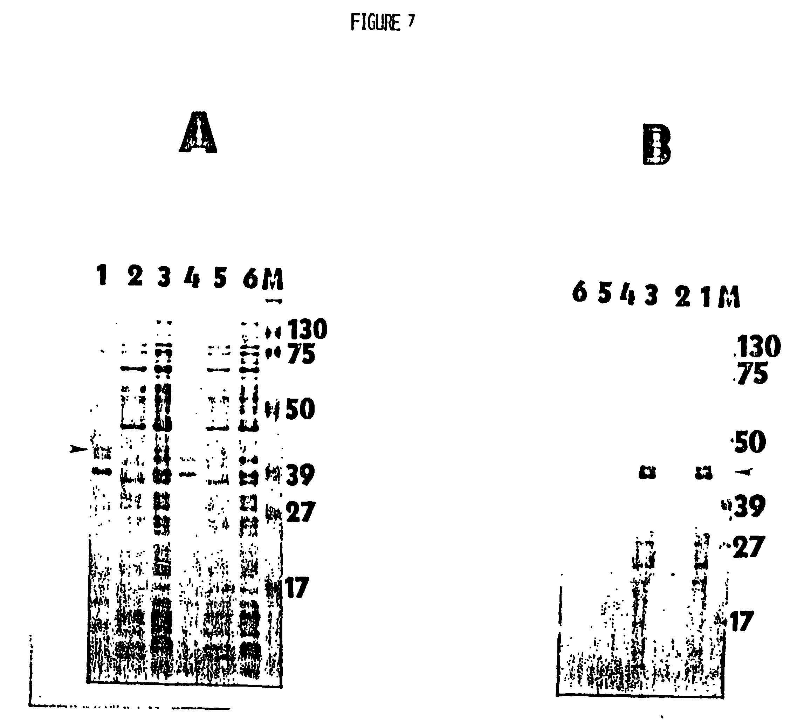

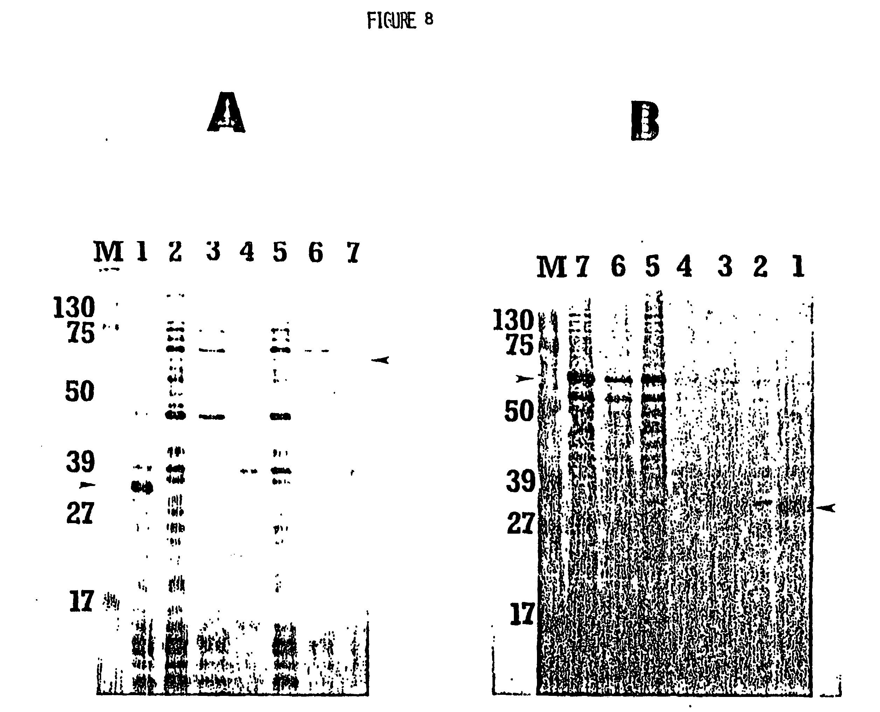

Whole cell lysates and the sonicated soluble and insoluble fractions of the cultures were analyzed and are illustrated in figures 7 and 8 for the pSD306 and pSD307 constructs, respectively.

Fig. 7 presents the expression of pSD306 prior to (T0) and two hours post (T2) induction, analyzed by Coomassie blue staining (Fig. 7 A) and immunoblotting (Fig. 7B). Samples were T0 whole cell lysate (lane 1); T0 sonicated soluble fraction (lane 2); T0 sonicated insoluble fraction (lane 3); T2 whole cell lysate (lane 4); T2 sonicated soluble fraction (lane 5); T2 sonicated insoluble fraction (lane 6); and BioRad prestained molecular weight markers (lane M). Fig. 7 demonstrates that pSD306 expressed a significant amount of the HIV-2 TMP at time T2, as indicated by the arrows on both the Coomassie stained gel and the immunoblot. This expressed protein is visible in both the whole cell lysate as well as the sonicated insoluble cell fraction of these cultures.

Similarly, Fig. 8 presents the expression of pSD307 prior to (T0) and two hours post (T2) induction, analyzed by Coomassie blue staining (Fig. 8 A) and immunoblotting (Fig. 8 B). Samples were pKRR955, T2 whole cell lysate (lane 1); pSD307, T0 whole cell lysate (lane 2), T0 sonicated soluble fraction (lane 3), T0 sonicated insoluble fraction (lane 4), T2 whole cell lysate (lane 5), T2 sonicated soluble fraction (lane 6), T2 sonicated insoluble fraction (lane 7); and BioRad prestained molecular weight markers (lane M). Fig. 8 demonstrates that pSD307 expressed a significant amount of the HIV-1 gag/HIV-2 TMP fusion protein at time T2, as indicated by the arrows on both the Coomassie stained gel and the immunoblot. This fusion protein is also visible in both the whole cell lysate and the sonicated insoluble fraction of these cultures. The HIV-1 gag fusion partner (lane 1), although present at higher levels than the HIV-1 gag/HIV-2 TMP fusion protein, showed lower immunoreactivity to HIV-2 specific antibodies.

### Example 3

### Diagnostic utility of synthetic DNA derived HIV proteins

The HIV specific proteins overexpressed in *E. coli* were purified using procedures known in the art. The proteins expressed at high levels were immunogenic and were recognized by antibodies produced in HIV-infected individuals (see figs. 7 and 8). The HIV specific proteins derived from *E. coli* can be utilized in several immunoassay configurations, as described in CIP application U.S. Serial No. 020,282, filed February 27, 1987 by Dawson et al., which is hereby incorporated by reference. The parent application is EPO 86116854.0 (December 4, 1986). In a preferred configuration, HIV specific proteins were coated on solid support and incubated with test samples. The virus specific antibodies present in the test sample recognized and were bound to the HIV proteins on the solid support. The HIV specific antibodies were quanfitated by the use of goat anti-human immunoglobulin conjugated to HRPO.

The HIV-1 exposed individuals were detected by the use of HIV-1 specific proteins, such as HIV-1 gp41 and HIV-1 p24 proteins derived by recombinant DNA techniques, described in the CIP application Serial No. 020,282. However, only 70 to 90% of the HIV-2 exposed individuals were detected using these HIV-1 specific proteins, due to cross reactivity between the two strains. The HIV-2 exposed individuals which were not detected using these HIV-1 specific proteins were detected using synthetic DNA derived HIV-2 proteins.

For example, the HIV-2 TMP fragment fused to CKS (pJC100) when supplemented to the recombinant HIV-1 proteins on the solid support described above significantly increased the detection of test samples containing HIV-2 antibodies as illustrated in Table 1, below.

**Table 1**

| | HIV-1 Test | HIV-1/HIV-2 Test |
|---|---|---|
| Samples Tested* | 127 | 127 |
| Non Reactive | 26 (20.47%) | 0 (0%) |
| Reactive | 101 (79.53%) | 127 (100%) |

| | | |
|---|---|---|
| * All 127 samples were confirmed positive for the presence of HIV-2 antibodies by western blot analysis using disrupted HIV-2 virus. | | |

Additionally, 3,411 normal blood donors were screened using the HIV-1/HIV-2 recombinant assay described above. The recombinant assay demonstrated a specificity of 99.77%, with only eight (0.23%) initial reactive and four (0.12%) repeat reactive samples.

### Example 4

### Differentiation of HIV-1 and HIV-2 infections

Frequently, individuals who have been exposed to HIV-2 have antibodies directed against epitopes on HIV-2 proteins which are also present on HIV-1 proteins. Likewise, individuals who have been exposed to HIV-1 have antibodies which cross-react with HIV-2 proteins. Because most of the cross-reactions are related to the gag gene products, the pJP100 protein and a recombinant protein from HIV-1 envelope protein (described in CIP Application Serial Number 020,282) were used to differentiate between individuals infected with HIV-1 and HIV-2.

Two independent enzyme-linked immunoassays were developed. Test 1 used HIV-1 recombinant proteins coated upon a solid phase. Test 2 used HIV-2 TMP (pJP100) coated upon a solid phase. Specimens from HIV seropositive individuals from the United States, Portugal or West Africa were tested for antibodies using these two tests. Endpoint titers were determined by diluting the specimens in normal human plasma and testing the dilutions. As illustrated in Table 2 below, specific tests using synthetic recombinant proteins can be effectively used to differentiate HIV-1 and HIV-2 infections.

**Table 2**

| Specimen | Test 1 Endpoint Titer | Test 2 Endpoint Titer |
|---|---|---|
| Chicago-AIDS-1 | 256 | <1 |
| Chicago-AIDS-2 | 512 | <1 |
| Chicago-AIDS-3 | 512 | <1 |
| Chicago-Asymptomatic-4 | 1024 | <1 |
| Chicago-Asymptomatic-5 | 2048 | <1 |
| Chicago-Asymptomatic-6 | 512 | <1 |
| West Africa-1 | <1 | 2048 |
| West Africa-2 | <1 | 64 |
| Portugal-1 | <1 | 512 |

Biological samples which are easily tested by the methods of the present invention include human and animal body fluids such as whole blood, serum, plasma, urine, saliva, stools, lymphocyte or cell culture preparations and purified and partially purified immunoglobulins. The polypeptides and fragments described herein can be used to determine the presence or absence of antibodies to HIV-1 and HIV-2 antigens by assay methods known to those skilled in the art, and for distinguishing between HIV-1 and HIV-2 infections.

One such assay involves:
a) coating a solid support with the polypeptides and polypeptide fragments disclosed herein;
b) contacting the coated solid support with the biological sample to form an antibody polypeptide complex;
c) removing unbound biological sample from the solid support;
d) contacting the complex on the solid support with a labeled immunoglobulin specific for the antibody; and
e) detecting the label to determine the presence or absence of HIV-2 antibodies in the biological sample.

A second assay method involves:
a) coating a solid support with the polypeptides and polypeptide fragments disclosed herein;
b) contacting the coated solid support with the biological sample and the homologous polypeptides conjugated to a label;
c) removing unbound biological sample and unbound labeled polypeptide; and
d) detecting the label to determine the presence or absence of HIV-2 antibodies in the biological sample.

Solid supports which can be used in such immunoassays include wells of reaction trays, test tubes, beads, strips, membranes, filters, microparticles or other solid supports which are well known to those skilled in the art. Enzymatic, radioisotopic, fluorescent, chemiluminescent and colloidal particle labels can be used in the aforementioned assays. Furthermore, hapten/labeled anti-hapten systems such as a biotin/labeled anti-biotin system can be utilized in the inventive assays. Both polyclonal and monoclonal antibodies are useful as reagents, and IgG as well as IgM class HIV antibodies may be used as solid support or labeled reagents.

It is evident from the foregoing examples that one skilled in the art could clone together specific subfragments of the synthetic genes constructed to generate new synthetic genes that would have the same characteristics as those illustrated herein. For example, the c-term gp120 subfragment, BS2-10 and subfragment 413-1 can be cloned together to produce synthetic gene products useful as diagnostic and therapeutic reagents for AIDS.

## Claims

1. A polypeptide comprising an amino acid sequence represented by the following:

MSLKIYSSAHGRHTRGVFVLGFLGFLATAGSAMGAASLTVSAQSRTLLAGIVQQQQLLDVVKRQQE
LTVWGTKNLQARVTAIEKYLQDQARLNSWGCAFRQVCHTTVPWVNDSLAPDWDNMTWQEWEKQVRYL
ISKSLEQAQIQQEKNMYELQKLNSWDIFGNWFDLTSWVKYIQYGVLIIVAVIALRIVIYVVQMLSRL
YRPVFSSPPGYIQQIHIHKDRGQPANEETEEDGGSNGGDRYWPWPIAYIHFLIRQLIRLLTRLYSIC
LSRSFLTLQLIYQNLRDWLRLRTAFLQYGCEWIQEAFQAAARATRETLAGACRGLWRVLERIGRGIL
RRIRQGAEIALLVPSSWQFGPG.

2. A polypeptide fragment of the polypeptide of Claim 1 comprising an amino acid sequence represented by the following:

YSSAHGRHTRGVFVLGFLGFLATAGSAMGAASLTVSAQSRTLLAGIVQQQQLLDVVKRQQELLRLTV
TKNLQARVTAIEKYLQDQARLNSWGCAFRQVCHTTVPW

3. The polypeptide of Claims 1 or 2 produced by E. coli.

4. A fusion polypeptide comprising a polypeptide as in one of Claims 1-2, in which the polypeptide is fused to a prokaryotic or eukaryotic protein.

5. The fusion polypeptide of Claim 4 wherein said prokaryotic or eukaryotic protein is the E. coli enzyme CKS.

6. A synthetic gene comprising a DNA sequence represented by tne following:

ATGAGCTTAAAGATCTACTCTTCCGCTCACGGCCGTCACACCCGTGGCGTTTTCGTTCTGGCTTCC
GCTTCCTGGCTACCGCGGGCTCCGCTATGGGCGCTGCTTCCCTGACCGTTTCCGCTCAGTCCCGTAC
GCTGGCTGGCATCGTTCAGCAGCAGCAGCAACTTCTAGACGTTGTTAAACGTCAGCAGGAGCTCCTG
CTGACCGTTTGGGGCACCAAAAACCTGCAGGCTCGTGTTACCGCTATCGAAAAATACCTGCAGGACC[?]
CTCGTCTGAATTCCTGGGGCTGCGCTTTCCGTCAGGTTTGCCACACCACCGTTCCATGGGTTAACGA[?]
CCTGGCTCCGGACTGGGACAACATGACCTGGCAGGAATGGGAAAAACAGGTTCGTTACCTGGAAGCT[?]
ATCTCCAAATCCCTGGAACAGGCTCAGATCCAGCAGGAAAAAAACATGTACGAACTGCAGAAACTGA[?]
CCTGGGATATCTTCGGCAACTGGTTCGACCTGACCTCCTGGGTTAAATATATCCAGTACGGCGTGCT[?]
CATCGTTGCTGTTATCGCTCTGCGTATCGTTATCTACGTAGTTCAGATGCTGTCCCGTCTGCGTAAA[?]
TACCGTCCGGTTTTCTCTTCCCCCCCGGGCTATATCCAGCAGATCCATATCCACAAAGACCGTGGCC[?]
CGGCTAACGAAGAAACCGAAGAAGACGGCGGATCCAACGGCGGCGACCGTTACTGGCCGTGGCCGAT[?]
TTATATCCACTTCCTGATCCGTCAGCTGATCCGTCTGCTGACCCGTCTATACTCCATCTGCCGTGAC[?]
CTGTCCCGTTCCTTCCTGACCCTGCAACTGATCTACCAGAACCTGCGTGACTGGCTGCGTCTGCGTA[?]
CTTTCCTGCAGTACGGCTGCGAATGGATTCAGGAAGCATTCCAAGCGGCCGCTCGTGCTACCCGTGA[?]
CCTGGCTGGCGCATGCCGTGGCCTGTGGCGTGTTCTGGAACGTATCGGCCGTGGTATCCTGGCTGTT[?]
CGTCGTATCCGTCAGGGCGCCGAAATCGCTCTGCTGGTACCAAGCTCATGGCAATTCGGGCCCGGT[?]

7. The synthetic gene of Claim 6 coding for the polypeptide of Claim 1.

8. A synthetic gene comprising a DNA sequence represented by the following:

TACTCTTCCGCTCACGGCCGTCACACCCGTGGCGTTTTCGTTCTGGGCTTCCTGGGCTTCCTGGCTAC
CGGGCTCCGCTATGGGCGCTGCTTCCCTGACCGTTTCCGCTCAGTCCCGTACCCTGCTGGCTGGCATC
TCAGCAGCAGCAGCAACTTCTAGACGTTGTTAAACGTCAGCAGGAGCTCCTGCGTCTGACCGTTTGGG
ACCAAAAACCTGCAGGCTCGTGTTACCGCTATCGAAAAATACCTGCAGGACCAGGCTCGTCTGAATTC
GGGGCTGCGCTTTCCGTCAGGTTTGCCACACCACCGTTCCATGG

9. The synthetic gene of Claim 8 coding for the polypeptide of Claim 2.

10. A fusion polypeptide comprising a polypeptide as in one of Claims 1-2, in which the polypeptide is fused to a HIV gag protein.

11. The fusion polypeptide of Claim 10 wherein said HIV gag protein is an HIV-1 gag protein comprising an amino acid sequence represented by the following:

DTGHSSOVSONYPIVONIOGOMVHOAISPRTLNAWVKVVEEKAFSPEVIPMFSALSEGATPODLNTML
VGGHOAAMOMLKETINEEAAEWDRVHPVHAGPIAPGOMREPRGSDIAGTTSTLOEQIGWMTNNPPIPV
IYKRWIILGLNKIVRMYSPTSILDIROGPKEPFRDYVDRFYKTLRAEQASOEVKNWMTETLLVQNANP
KTILKALGPAATLEEMMTACQGVGGPGHKARVLAEAMSQVTNTATIMMQRGNFRNQRKMVKCFNCGKE
TARNCRA

12. A method for detecting antibodies to HIV antigens in an individual which comprises the steps of:
a) obtaining a sample of a body fluid which has been obtained from the individual:
b) incubating said body fluid with said polypeptide of Claims 1 or 2;
c) incubating said body fluid with a labeled antibody to immunoglobulin; and
d) detecting said label and determining therefrom the presence or absence of antibodies to HIV antigens.

13. A method for detecting antibodies to HIV antigens in an individual which comprises the steps of:
a) obtaining a sample of a body fluid which has been obtained from the individual:
: b) incubating said body fluid with said polypeptide of Claims 1 or 2;
c) incubating said body fluid with a labeled antigen; and
d) detecting said label and determining therefrom the presence or absence of antibodies to HIV antigens.

## Patentansprüche

1. Ein Polypeptid, das eine Aminosäuresequenz umfasst, dargestellt durch folgende:

MSLKIYSSAHGRHTRGVFVLGFLGFLATAGSAMGAASLTVSAQSRTLLAGIVQQQQQLLDVVKRQQEL
LTVWGTKNLQARVTAIEKYLQDQARLNSWGCAFRQVCHTTVPWVNDSLAPDWDNMTWQEWEKQVRYLE
ISKSLEQAQIQQEKNMYELQKLNSWDIFGNWFDLTSWVKYIQYGVLIIVAVIALRIVIYVVQMLSRLR
YRPVFSSPPGYIQQIHIHKDRGQPANEETEEDGGSNGGDRYWPWPIAYIHFLIRQLIRLLTRLYSICR
LSRSFLTLQLIYQNLRDWLRLRTAFLQYGCEWIQEAFQAAARATRETLAGACRGLWRVLERIGRGILA
RRIRQGAEIALLVPSSWQFGPG.

2. Ein Polypeptid-Fragment des Polypeptids von Anspruch 1, das eine Aminosäuresequenz umfasst, dargestellt durch folgende:

YSSAHGRHTRGVFVLGFLGFLATAGSAMGAASLTVSAQSRTLLAGIVQQQQQLLDVVKRQQELLRLTVWG
TKNLQARVTAIEKYLQDQARLNSWGCAFRQVCHTTVPW

3. Das Polypeptid von Ansprüchen 1 oder 2, produziert durch E. coli.

4. Ein Fusions-Polypeptid, das ein Polypeptid umfasst, wie in einem der Ansprüche 1 bis 2, worin das Polypeptid an ein prokaryotisches oder eukaryotisches Protein fusioniert ist.

5. Das Fusions-Polypeptid von Anspruch 4, worin das prokaryotische oder eukaryotische Protein das E. coli Enzym CKS ist.

6. Ein synthetisches Gen, das eine DNA-Sequenz umfasst, dargestellt durch folgende:

ATGAGCTTAAAGATCTACTCTTCCGCTCACGGCCGTCACACCCGTGGCGTTTTCGTTCTGGCTTCCTGG
GCTTCCTGGCTACCGCGGGCTCCGCTATGGGCGCTGCTTCCCTGACCGTTTCCGCTCAGTCCCGTACCCT
GCTGGCTGGCATCGTTCAGCAGCAGCAGCAACTTCTAGACGTTGTTAAACGTCAGCAGGAGCTCCTGCGT
CTGACCGTTTGGGGCACCAAAAACCTGCAGGCTCGTGTTACCGCTATCGAAAAATACCTGCAGGACCAGG
CTCGTCTGAATTCCTGGGGCTGCGCTTTCCGTCAGGTTTGCCACACCACCGTTCCATGGGTTAACGATTC
CCTGGCTCCGGACTGGGACAACATGACCTGGCAGGAATGGGAAAAACAGGTTCGTTACCTGGAAGCTAAC
ATCTCCAAATCCCTGGAACAGGCTCAGATCCAGCAGGAAAAAAACATGTACGAACTGCAGAAACTGAACT
CCTGGGATATCTTCGGCAACTGGTTCGACCTGACCTCCTGGGTTAAATATATCCAGTACGGCGTGCTCAT
CATCGTTGCTGTTATCGCTCTGCGTATCGTTATCTACGTAGTTCAGATGCTGTCCCGTCTGCGTAAAGGC
TACCGTCCGGTTTTCTCTTCCCCCCCGGGCTATATCCAGCAGATCCATATCCACAAAGACCGTGGCCAGC
CGGCTAACGAAGAAACCGAAGAAGACGGCGGATCCAACGGCGGCGACCGTTACTGGCCGTGGCCGATCGC
TTATATCCACTTCCTGATCCGTCAGCTGATCCGTCTGCTGACCCGTCTATACTCCATCTGCCGTGACCTG
CTGTCCCGTTCCTTCCTGACCCTGCAACTGATCTACCAGAACCTGCGTGACTGGCTGCGTCTGCGTACCG
CTTTCCTGCAGTACGGCTGCGAATGGATTCAGGAAGCATTCCAAGCGGCCGCTCGTGCTACCCGTGAAAC
CCTGGCTGGCGCATGCCGTGGCCTGTGGCGTGTTCTGGAACGTATCGGCCGTGGTATCCTGGCTGTTCCG
CGTCGTATCCGTCAGGGCGCCGAAATCGCTCTGCTGGTACCAAGCTCATGGCAATTCGGCCCGGGTAA

7. Das synthetische Gen von Anspruch 6, das für das Polypeptid von Anspruch 1 kodiert.

8. Ein synthetisches Gen, das eine DNA-Sequenz umfasst, die durch folgende dargestellt ist:

TACTCTTCCGCTCACGGCCGTCACACCCGTGGCGTTTTCGTTCTGGGCTTCCTGGGCTTCCTGGCTACC
CGGGCTCCGCTATGGGCGCTGCTTCCCTGACCGTTTCCGCTCAGTCCCGTACCCTGCTGGCTGGCATCG
TCAGCAGCAGCAGCAACTTCTAGACGTTGTTAAACGTCAGCAGGAGCTCCTGCGTCTGACCGTTTGGGG
ACCAAAAACCTGCAGGCTCGTGTTACCGCTATCGAAAAATACCTGCAGGACCAGGCTCGTCTGAATTCC
GGGGCTGCGCTTTCCGTCAGGTTTGCCACACCACCGTTCCATGG

9. Das synthetische Gen von Anspruch 8, das für das Polypeptid von Anspruch 2 kodiert.

10. Ein Fusions-Polypeptid, das ein Polypeptid wie in einem der Ansprüche 1 bis 2 umfasst, worin das Polypeptid an ein HIV-gag-Protein fusioniert ist.

11. Das Fusions-Polypeptid von Anspruch 10, worin das HIV-gag-Protein ein HIV-1 gag-Protein ist, das eine Aminosäuresequenz umfasst, die durch folgende dargestellt ist:

DTGHSSQVSQNYPIVQNIQGQMVHQAISPRTLNAWVKVVEEKAFSPEVIPMFSALSEGATPQDLNTML
VGGHQAAMQMLKETINEEAAEWDRVHPVHAGPIAPGQMREPRGSDIAGTTSTLQEQIGWMTNNPPIPV
IYKRWIILGLNKIVRMYSPTSILDIRQGPKEPFRDYVDRFYKTLRAEQASQEVKNWMTETLLVQNANP
KTILKALGPAATLEEMMTACQGVGGPGHKARVLAEAMSQVTNTATIMMQRGNFRNQRKMVKCFNCGKE
TARNCRA

12. Ein Verfahren zum Detektieren von Antikörpern gegen HIV-Antigene in einem Individuum, das die folgenden Schritte umfasst:
a) Erhalten einer Probe aus einem Körper-Fluid, das aus dem Individuum erhalten wurde;
b) Inkubieren des Körper-Fluids mit dem Polypeptid von Ansprüchen 1 oder 2;
c) Inkubieren des Körper-Fluids mit einem markierten Antikörper gegen Immunglobulin; und
d) Detektieren des Markers und daraus die Anwesenheit oder die Abwesenheit von Antikörpern gegen HIV-Antigene bestimmen.

13. Ein Verfahren zur Detektion von Antikörpern gegen HIV-Antigene in einem Individuum, welches die folgenden Schritte umfasst:
a) Erhalten einer Probe eines Körper-Fluids, das aus dem Individuum erhalten wurde;
b) Inkubieren des Körper-Fluids mit dem Polypeptid von Ansprüchen 1 oder 2;
c) Inkubieren des Körper-Fluids mit einem markierten Antigen; und
d) Detektieren des Markers und daraus die Anwesenheit oder Abwesenheit von Antikörpern gegen HIV-Antigene bestimmen.

## Revendications

1. Polypeptide comprenant une séquence d'aminoacides représentée par la séquence suivante:

MSLKIYSSAHGRHTRGVFVLGFLGFLATAGSAMGAASLTVSAQSRTLLAGIVQQQQQLLDVVKRQ[?]
LTVWGTKNLQARVTAIEKYLQDQARLNSWGCAFRQVCHTTVPWVNDSLAPDWDNMTWQEWEKQVR[?]
ISKSLEQAQIQQEKNMYELQKLNSWDIFGNWFDLTSWVKYIQYGVLIIVAVIALRIVIYVVQMLS[?]
YRPVFSSPPGYIQQIHIHKDRGQPANEETEEDGGSNGGDRYWPWPIAYIHFLIRQLIRLLTRLYS[?]
LSRSFLTLQLIYQNLRDWLRLRTAFLQYGCEWIQEAFQAAARATRETLAGACRGLWRVLERIGRG[?]
RRIRQGAEIALLVPSSWQFGPG.

2. Fragment polypeptidique du polypeptide de la revendication 1, comprenant une séquence d'aminoacides représentée par la séquence suivante:

YSSAHGRHTRGVFVLGFLGFLATAGSAMGAASLTVSAQSRTLLAGIVQQQQQLLDVVKRQQELLRL
TKNLQARVTAIEKYLQDQARLNSWGCAFRQVCHTTVPW

3. Polypeptide selon la revendication 1 ou 2, produit par *E. coli*.

4. Polypeptide de fusion comprenant un polypeptide selon l'une des revendications 1-2, dans lequel le polypeptide est fusionné avec une protéine procaryote ou eucaryote.

5. Polypeptide de fusion selon la revendication 4, dans lequel ladite protéine procaryote ou eucaryote est l'enzyme CKS d'*E*. *coli.*

6. Gène synthétique comprenant une séquence d'ADN représentée par la séquence suivante:

ATGAGCTTAAAGATCTACTCTTCCGCTCACGGCCGTCACACCCGTGGCGTTTTCGTTCTGGGCTT[?]
GCTTCCTGGCTACCGCGGGCTCCGCTATGGGCGCTGCTTCCCTGACCGTTTCCGCTCAGTCCCGT[?]
GCTGGCTGGCATCGTTCAGCAGCAGCAGCAACTTCTAGACGTTGTTAAACGTCAGCAGGAGCTCC[?]
CTGACCGTTTGGGGCACCAAAAACCTGCAGGCTCGTGTTACCGCTATCGAAAAATACCTGCAGG[?]
CTCGTCTGAATTCCTGGGGCTGCGCTTTCCGTCAGGTTTGCCACACCACCGTTCCATGGGTTAAC[?]
CCTGGCTCCGGACTGGGACAACATGACCTGGCAGGAATGGGAAAAACAGGTTCGTTACCTGGAAG[?]
ATCTCCAAATCCCTGGAACAGGCTCAGATCCAGCAGGAAAAAAACATGTACGAACTGCAGAAACT[?]
CCTGGGATATCTTCGGCAACTGGTTCGACCTGACCTCCTGGGTTAAATATATCCAGTACGGCGTG[?]
CATCGTTGCTGTTATCGCTCTGCGTATCGTTATCTACGTAGTTCAGATGCTGTCCCGTCTGCGTA[?]
TACCGTCCGGTTTTCTCTTCCCCCCCGGCTATATCCAGCAGATCCATATCCACAAAGACCGTGG[?]
CGGCTAACGAAGAAACCGAAGAAGACGGCGGATCCAACGGCGGCGACCGTTACTGGCCGTGGCCG[?]
TTATATCCACTTCCTGATCCGTCAGCTGATCCGTCTGCTGACCCGTCTATACTCCATCTGCCGTG[?]
CTGTCCCGTTCCTTCCTGACCCTGCAACTGATCTACCAGAACCTGCGTGACTGGCTGCGTCTGCG[?]
CTTTCCTGCAGTACGGCTGCGAATGGATTCAGGAAGCATTCCAAGCGGCCGCTCGTGCTACCCGT[?]
CCTGGCTGGCGCATGCCGTGGCCTGTGGCGTGTTCTGGAACGTATCGGCCGTGGTATCCTGGCTG[?]
CGTCGTATCCGTCAGGGCGCCGAAATCGCTCTGCTGGTACCAAGCTCATGGCAATTCGGCCCGG[?]

7. Gène synthétique selon la revendication 6, codant pour le polypeptide de la revendication 1.

8. Gène synthétique comprenant une séquence d'ADN représentée par la séquence suivante:

TACTCTTCCGCTCACGGCCGTCACACCCGTGGCGTTTTCGTTCTGGGCTTCCTGGGCTTCCTGGCT
CGGGCTCCGCTATGGGCGCTGCTTCCCTGACCGTTTCCGCTCAGTCCCGTACCCTGCTGGCTGGCA
TCAGCAGCAGCAGCAACTTCTAGACGTTGTTAAACGTCAGCAGGAGCTCCTGCGTCTGACCGTTTG
ACCAAAAACCTGCAGGCTCGTGTTACCGCTATCGAAAAATACCTGCAGGACCAGGCTCGTCTGAAT
GGGGCTGCGCTTTCCGTCAGGTTTGCCACACCACCGTTCCATGG

9. Gène synthétique selon la revendication 8, codant pour le polypeptide de la revendication 2.

10. Polypeptide de fusion comprenant un polypeptide selon l'une des revendications 1-2, dans lequel le polypeptide est fusionné avec une protéine gag de VIH.

11. Polypeptide de fusion selon la revendication 10, dans lequel ladite protéine gag de VIH est une protéine gag de VIH-1 comprenant une séquence d'aminoacides représentée par la séquence suivante:

DTGHSSOVSONYPIVONIOGOMVHOAISPRTLNAWVKVVEEKAFSPEVIPMFSALSEGATPODLNT
VGGHOAAMOMLKETINEEAAEWDRVHPVHAGPIAPGOMREPRGSDIAGTTSTLOEQIGWMTNNPPI
IYKRWIILGLNKIVRMYSPTSILDIROGPKEPFRDYVDRFYKTLRAEQASOEVKNWMTETLLVQNA
KTILKALGPAATLEEMMTACQGVGGPGHKARVLAEAMSQVTNTATIMMQRGNFRNQRKMVKCFNCG
TARNCRA

12. Procédé de détection d'anticorps dirigés contre des antigènes de VIH chez un individu, qui comprend les étapes selon lesquelles:
a) on obtient un échantillon d'un liquide corporel qui a été obtenu à partir de l'individu;
b) on met ledit liquide corporel à incuber avec ledit polypeptide des revendications 1 ou 2;
c) on met ledit liquide corporel à incuber avec un anticorps anti-immunoglobuline marqué; et
d) on détecte ledit marqueur et on en déduit la présence ou l'absence d'anticorps dirigés contre des antigènes de VIH.

13. Procédé de détection d'anticorps dirigés contre des antigènes de VIH chez un individu, qui comprend les étapes selon lesquelles:
a) on obtient un échantillon d'un liquide corporel qui a été obtenu à partir de l'individu;
b) on met ledit liquide corporel à incuber avec ledit polypeptide des revendications 1 ou 2;
c) on met ledit liquide corporel à incuber avec un antigène marqué; et
d) on détecte ledit marqueur et on en déduit la présence ou l'absence d'anticorps dirigés contre des antigènes de VIH.
